# EUROPEAN PATENT APPLICATION

(11) **EP 2 218 470 A1**
(43) Date of publication of application: **18.08.2010**
(21) Application number: 08847738.5
(22) Date of filing: 27.10.2008
(51) Int. Cl.: A61M 1/14

(54) **HEMODIALYSIS APPARATUS**

(30) Priority: 06.11.2007 JP 2007288094
(71) Applicant: JMS Co., Naka-ku, Hiroshima-shi Hiroshima 730-8652 (JP)
(72) Inventor: MASAOKA, Katsunori, Hiroshima-shi Hiroshima 730-8652 (JP)
(74) Representative: Vossius & Partner
(86) International application number: PCT/JP2008/069439
(87) International publication number: WO 2009/060741

(57) **Abstract**

Provided is a hemodialyzer for preventing beforehand any medical accident such as air inclusion or blood loss during a medical treatment by detecting even a minute connection failure, if any. The hemodialyzer includes a hemodialyzer (D), a dialysate supply line (L1), a dialysate discharge line (L2), an artery side blood line (L3), a vein side blood line (L4), first fluid feeding means (P1), second fluid feeding means (P2), third fluid feeding means (P3), a blood pump (P4), a vein side chamber (C_{V}), an overflow line (L5), a venous pressure monitor line (L6), dialysate pressure measuring means (M_{T}), venous pressure measuring means (M_{V}), clamps (CL_{L4}, CL_{L5}), control means (G1) that closes the clamp (CL_{L5}) when a predetermined given period of time has elapsed from the start of priming, and operates the third fluid feeding means (P3) in a reverse filtration direction or in a fluid removal direction, and then opens the clamp (CL_{L5}) when a predetermined given period of time has elapsed, and judging means (J1) that compares a pressure variation amount in the dialysate pressure or the venous pressure for a period of time when the clamp (CL_{L5}) is closed with a predetermined reference value to judge normality of a circuit connection.

## Description

### Technical Field

The present invention relates to a hemodialysis apparatus that is used for a medical treatment with an extracorporeal circulation of blood such as hemodialysis, continuous hemofiltration, or plasmapheresis, and more particularly, to a hemodialysis apparatus that verifies a sealed degree of an extracorporeal circulation circuit in which blood circulates, to thereby judge normality of a circuit connection so as to prevent beforehand any medical accident such as air inclusion or blood loss during the medical treatment, in a preliminary phase for starting a medical treatment or in an imminent phase for starting the medical treatment.

### Background Art

A hemodialysis apparatus is a type of medical equipment that extracorporeally circulates the blood of a patient with renal failure or a drug intoxicated patient to perform blood purification. The hemodialysis apparatus generally includes three portions of (1) a hemodialyzer D that brings blood into contact with a dialysate through a semipermeable membrane to purify blood, (2) a dialysate supply/discharge system mainly including a dialysate supply line L1 that supplies the dialysate to the hemodialyzer D, and a dialysate discharge line L2 that discharges the dialysate from the hemodialyzer D, and (3) a blood circuit mainly including an artery side blood line L3 that allows the blood drawn from the patient to flow into the hemodialyzer D, and a vein side blood line L4 that returns the blood drained from the hemodialyzer D to the patient.

In the dialysate supply/discharge system, the dialysate supply line L1 and the dialysate discharge line L2 are connected to the hemodialyzer D, and first fluid feeding means P1 that feeds the dialysate to the hemodialyzer D is connected to the dialysate supply line L1. On the other hand, second fluid feeding means P2 that absorbs the dialysate from the hemodialyzer D is connected to the dialysate discharge line L2. Further, any one or both of the first fluid feeding means P1 and the second fluid feeding means P2 are formed with a bypass line that communicates an upstream side thereof with a downstream side thereof. The bypass line is provided with third fluid feeding means P3 that moves the dialysate into the blood circuit by reverse filtration through the hemodialyzer D, and removes the blood within the hemodialyzer D.

The blood circuit mainly includes two circuits of a vein side blood circuit and an artery side blood circuit. In the former vein side blood circuit, the vein side blood line L4 is connected to the hemodialyzer D, a vein side chamber C_{V} is connected to the vein side blood line L4, and an overflow line L5 is connected to the vein side chamber C_{V}. In the latter artery side blood circuit, the artery side blood line L3 is connected to the hemodialyzer D, and a blood pump P4 that circulates the blood is connected to the artery side blood line L3. Further, an artery side chamber C_{A} may be connected to the artery side blood line L3.

Further, dialysate pressure measuring means M_{T} that measures a dialysate pressure which is a fluid pressure of the dialysate flowing in the dialysate discharge line L2 is connected to the dialysate discharge line L2, and venous pressure measuring means M_{V} that means a venous pressure which is a pressure within the vein side blood line L4 is connected to a venous pressure monitor line L6 connected to the vein side chamber C_{V}.

As described above, the hemodialysis apparatus is made up of an extremely large number of apparatus goods, and the blood drawn from the patient body, and the blood that has been purified by the extracorporeal circulation circulate inside the hemodialysis apparatus. Therefore, if a connection failure occurs in the extracorporeal circulation circuit, there is a fear that air is sucked into the extracorporeal circulation circuit to cause the air inclusion into the patient body, or conversely the blood in the extracorporeal circulation circuit is discharged out of the extracorporeal circulation circuit to induce a serious accident such as the loss of consciousness and decease due to blood loss.

Accordingly, in conducting a medical treatment using the hemodialysis apparatus made up of such a large number of apparatus goods, a healthcare professional must pay full attention to the normality of the extracorporeal circulation circuit not only before starting a medical treatment such as priming but also during the medical treatment. Further, in order to suppress occurrence of accidents as much as possible, an extremely sensitive handling such that clamps or forceps are doubled is required.

For that reason, in order to provide a safe dialysis treatment with no accident to the patient, and in order to reduce a physical and mental strain on the healthcare professional, there have been conventionally proposed several methods for detecting a connection failure of medical equipment such as the hemodialysis apparatus (for example, see Patent Document 1).

The detecting method disclosed in Patent Document 1 is a method for detecting the connection failure of a circuit in a blood purification device including at least a hemodialyzer D, an artery side blood circuit, a vein side blood circuit, a dialysate supply/discharge system, a pressure measurement line that is connected to at least one of the artery side blood circuit, the vein side blood circuit, and the dialysate supply/discharge system, pressure measuring means connected to the pressure measurement line, and fluid supply means connected to a terminal side with respect to the pressure measurement line.

The principle of the above-mentioned detecting method is based on such a technical knowledge that the connection failure of the circuit can be simply detected by detecting a pressure decrease of a priming fluid because a clean normal saline is caused to flow from a terminal of the vein side blood circuit as the priming fluid in priming a fluid flow passage. In order to realize this, in the above-mentioned detecting method, the fluid supply means is arranged at a position higher in level than the pressure measuring means, and after circulation of a fluid in the artery side blood circuit and the dialysate supply/discharge system is blocked, the pressure decrease between the fluid supply means and the pressure measuring means is detected by the pressure measuring means to detect the connection failure of the circuit.

However, a pressure that can be applied by arranging the fluid supply means at the position higher in level than the pressure measuring means is about 150 mmHg at a maximum. For that reason, in the above-mentioned detecting method, it appears that the circuits are normally connected, but a slight gap occurs in the circuit connection portion, and a so-called slow leak in which a fluid is leaked from the gap extremely bit by bit cannot be detected.
Patent Document 1: JP 2005-218709 A

### Disclosure of the Invention

### Problems to be solved by the Invention

A problem to be solved by the present invention is to provide a hemodialysis apparatus that detects, in a preliminary phase for starting a medical treatment or in an imminent phase for starting the medical treatment, a minute connection failure that cannot be detected through visual inspection by a healthcare professional, which is represented by a so-called slow leak, so as to prevent beforehand any medical accident such as air inclusion or blood loss during the medical treatment.

### Means for solving the Problems

The inventor of the present invention has obtained the following technical knowledge as a result of repeating various experimental studies and logical studies for solving the problem.

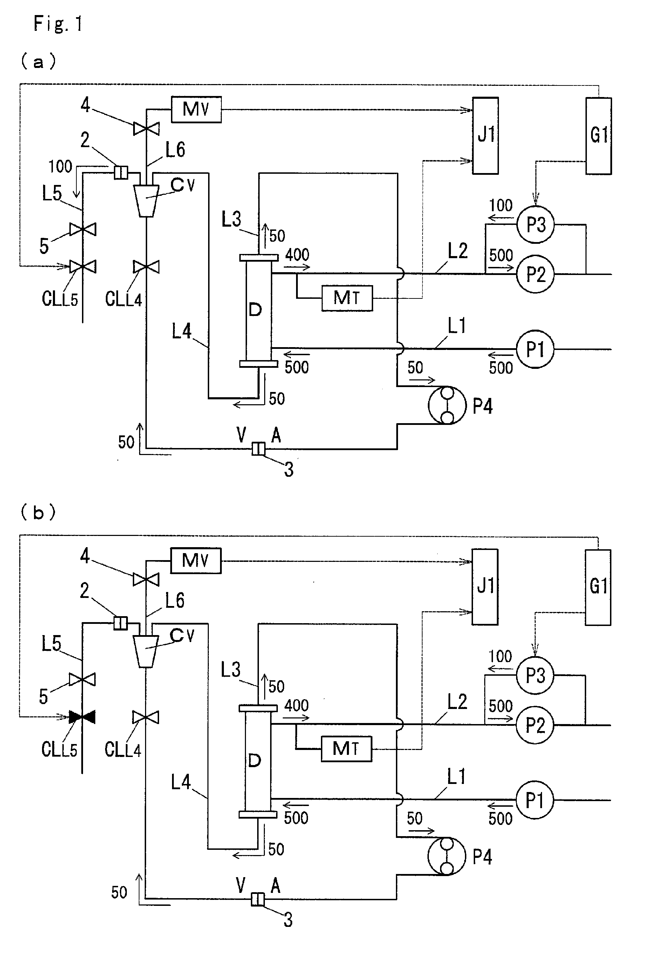

FIGS. 1 are diagrams illustrating a hemodialysis apparatus according to a first embodiment of the present invention, of which FIG. 1(a) is a diagram illustrating an operating state since the start of priming until a given period of time elapses, and FIG. 1(b) is a diagram illustrating an example of the subsequent operating state.
It should be noted that numerical numbers illustrated in FIGS. 1 indicate flow rates of a dialysate flowing in the respective lines when the circuit connection is normal, and arrows indicate directions along which the dialysate flows. Hereinafter, priming is described briefly with reference to FIG. 1(a).

Priming is to generally wash an extracorporeal circulation circuit before the dialysis treatment starts by the aid of a normal saline. However, in the hemodialysis apparatus according to the present invention, the extracorporeal circulation circuit is washed by the aid of not the normal saline but the dialysate.
As an example of priming with the dialysate, as illustrated in FIG. 1(a), third fluid feeding means P3 is operated at the flow rate of 100 ml/min in a reverse filtration direction in a state where first fluid feeding means P1 and second fluid feeding means P2 are operated at 500 ml/min. As a result, the flow rate of the dialysate pushed into a hemodialyzer D is larger than the flow rate pulled therefrom by 100 ml/min. Therefore, a reverse filtration phenomenon that the dialysate flowing outside a hollow fiber is pushed into the inside of the hollow fiber due to a difference in flow rate occurring inside the hemodialyzer D. The hemodialysis apparatus according to the present invention which is exemplified in FIGS. 1 washes the hemodialyzer D through the reverse filtration.

Further, because a blood pump P4 is operated at 50 ml/min in an inverse rotation direction (clockwise) in a state where the reverse filtration is proceeding, the dialysate pushed into the hollow fiber within the hemodialyzer D by reverse filtration flows into an artery side blood line L3 at 50 ml/min which is the same flow rate as the flow rate of the blood pump P4, and also flows in a vein side blood line L4 at the flow rate of 50 ml/min. The flow rate within the vein side blood line L4 is a value obtained by subtracting the flow rate 50 ml/min of the dialysate flowing in the artery side blood line L3 from 100 ml/min which is a difference between the flow rate 500 ml/min of the dialysate pushed into the hemodialyzer D and the flow rate 400 ml/min of the dialysate pulled from the hemodialyzer D.

Then, the dialysate that has washed the artery side blood line L3 and the vein side blood line L4 join together in a vein side chamber C_{V}, and is finally discharged from an overflow line L5 connected to the vein side chamber C_{V}. In this way, the dialysate pushed into the hollow fiber within the hemodialyzer D through reverse filtration flows into the artery side blood line L3 and the vein side blood line L4 to wash the artery side blood line L3 and the vein side blood line L4. The rough operation of priming by the aid of the dialysate is described above.

(A) Plural technical findings obtained by the inventor of the present invention with respect to the judgment of the normality by using the above-mentioned priming process are described below.

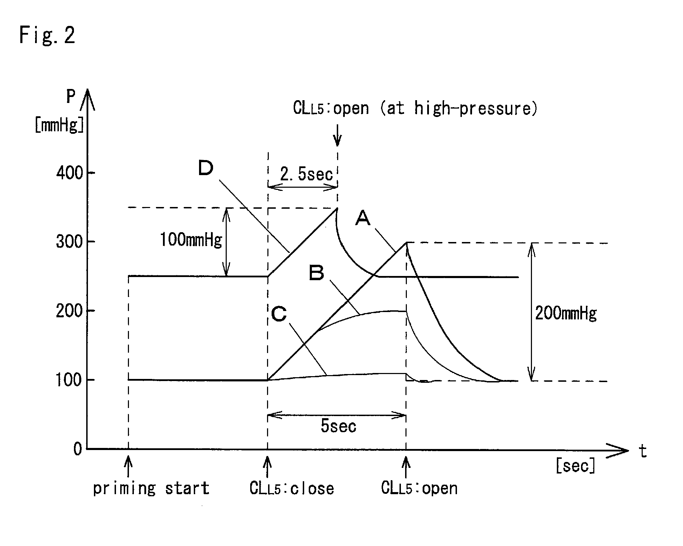

FIG. 2 is a diagram schematically illustrating an example of a judgment principle using the priming process, and illustrates a transition of the dialysate pressure measured by dialysate pressure measuring means M_{T} disposed in a dialysate discharge line L2.
A line A in the figure illustrates the transition of the dialysate pressure when the circuit connection is normal, a line B illustrates the transition of the dialysate pressure when a slight gap occurs in the connection portion of the hemodialyzer D and the artery side blood line L3, and a line C illustrates the transition of the dialysate pressure when an apparent connection failure that can be detected through visual inspection occurs in the connection portion of the hemodialyzer D and the artery side blood line L3.

As illustrated in FIGS. 2 and 1(b), when a predetermined given period of time has elapsed since the start of priming, a clamp CL_{L5} disposed in the overflow line L5 is closed, and the operation of the third fluid feeding means P3 that has been operated in the reverse filtration direction is continued.
With the above-mentioned operation, when the circuit connection is normal, the dialysate that has been discharged from the overflow line L5 during priming has nowhere to go, and the dialysate pressures in both of the artery side blood line L3 and the vein side blood line L4 increase, and accordingly the fluid pressure of the dialysate flowing in the dialysate discharge line L2 also increases. This is verified by a real machine as indicated by the line A illustrated in FIG. 2. For example, it can be verified that the dialysate pressure increases 200 mmHg within 5 sec when the clamp CL_{L5} is closed, under the condition illustrated in FIG. 1(b).

On the other hand, when a slight gap occurs in the connection portion between the hemodialyzer D and the artery side blood line L3, the flow rate of the dialysate flowing in the vein side blood line L4 is reduced by the amount of dialysate leaked from the gap. Therefore, the fluid pressure of dialysate flowing in the dialysate discharge line L2 does not increase as compared with the normal state. This is verified by a real machine as indicated by the line B illustrated in FIG. 2.
Further, when an apparent connection failure that can be detected through visual inspection occurs in the connection portion between the hemodialyzer D and the artery side blood line L3, the dialysate flows out from that portion, and the dialysate hardly flows in the artery side blood line L3. Therefore, the fluid pressure of the dialysate flowing in the dialysate discharge line L2 hardly varies. This is verified by a real machine as indicated by the line C illustrated in FIG. 2.

From the above-mentioned verification results, in the hemodialysis apparatus in which the normality of the circuit connection has been verified, a pressure variation amount in the dialysate pressure during the above-mentioned operation is measured and recorded under each of the priming conditions in advance, and then set as a reference value in advance. As a result, in the hemodialysis apparatus whose normality is to be verified, a pressure variation amount in the dialysate pressure during the above-mentioned operation is measured and compared with the reference values so as to judge the normality of the circuit connection. This is one of the technical knowledge obtained by the inventor of the present invention.

(B) Further, the inventor of the present invention has verified, through a real machine, that the venous pressure measured by the venous pressure measuring means M_{V} disposed in the venous pressure monitor line L6 exhibits the same transition as that of the dialysate pressure measured by the dialysate pressure measuring means M_{T} if the circuit connection is normal when the above-mentioned operation is conducted, although not illustrated. This is also one of the technical knowledge obtained by the inventor of the present invention with respect to the judgment of the normality by using the priming process.
Accordingly, in the hemodialysis apparatus in which the normality of the circuit connection has been verified, a pressure variation amount in the dialysate pressure or the venous pressure when the clamp CL_{L5} is closed and the third fluid feeding means P3 is operated in the reverse filtration direction, when a predetermined period of time has elapsed since the start of priming, is measured and recorded under each of the priming conditions in advance, and set as the reference value in advance. As a result, in the hemodialysis apparatus whose normality is to be verified, a pressure variation amount in the dialysate pressure or the venous pressure during the above-mentioned operation is measured and compared with the reference value so as to judge the normality of the circuit connection.

(C) The above-mentioned operation for judging the normality of the circuit connection described with reference to FIGS. 1 and 2 is an operation for closing the clamp CL_{L5} and continuing the operation of the third feeding means P3 that have been operated in the reverse filtration direction for a given period of time. Alternatively, as illustrated in FIG. 3, the normality of the circuit connection can be judged by operating the third fluid feeding means P3 in the fluid removal direction.

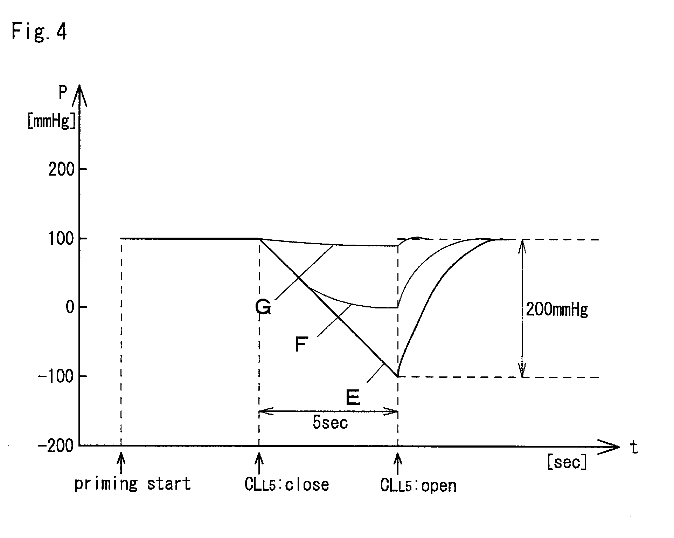

FIG. 4 is a diagram schematically illustrating an example of the judgment principle, and illustrates the transition of the dialysate pressure measured by the dialysate pressure measuring means M_{T} disposed in the dialysate discharge line L2.
A line E in the figure illustrates a transition of the dialysate pressure when the circuit connection is normal, a line F illustrates a transition of the dialysate pressure when a slight gap occurs in the connection portion between the hemodialyzer D and the artery side blood line L3, and a line G illustrates a transition of the dialysate pressure when an apparent connection failure that can be detected through visual inspection occurs in the connection portion between the hemodialyzer D and the artery side blood line L3.

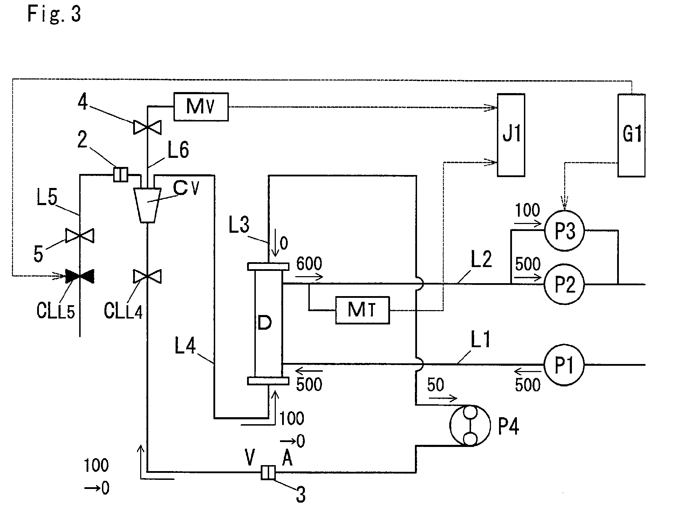

As illustrated in FIGS. 4 and 3, the clamp CL_{L5} is closed when a predetermined given period of time has elapsed since the start of priming, and the third fluid feeding means P3 that has been operated in the reverse filtration direction during priming is operated in the fluid removal direction.
With the above-mentioned operation, when the circuit connection is normal, the flow rate of the dialysate pushed into the hemodialyzer D is smaller than the pulled flow rate by 100 ml/min. Therefore, the dialysate pressures in both of the artery side blood line L3 and the vein side blood line L4 decrease, and accordingly the fluid pressure of the dialysate flowing in the dialysate discharge line L2 also decreases. This is verified by a real machine as indicated by the line E illustrated in FIG. 4. For example, it can be verified that the dialysate pressure decreases 200 mmHg within 5 sec when the clamp CL_{L5} is closed under the condition illustrated in FIG. 3.
It should be noted that numerical numbers illustrated in FIG. 3 indicate flow rates of the dialysate flowing in the respective lines when the circuit connection is normal, and arrows indicate directions along which the dialysate flows. As described above, when the circuit connection is normal, although the flow rate of the dialysate pushed into the hemodialyzer D should be smaller than the pull flow rate by 100 ml/min by the fluid removal operation due to the third fluid feeding means P3, the hemodialyzer D attempts to pull the dialysate of 100 ml/min which is a difference between the above-mentioned flow rates from the artery side blood line L3 or the vein side blood line L4. However, because the blood pump P4 is operated at 50 ml/min in the reverse rotation direction (clockwise direction), the dialysate cannot be pulled from the artery side blood line L3 (0 ml/min illustrated on an upper side of the hemodialyzer D). For that reason, the hemodialyzer D pulls the dialysate of 100 ml/min from the vein side blood line L4. However, because the dialysate filled within the vein side blood line L4 by priming is limited, the flow rate finally comes to 0 ml/min (100→0 ml/min illustrated on a lower side of the hemodialyzer D).

On the other hand, in the case where a slight gap occurs in the connection portion between the hemodialyzer D and the artery side blood line L3, the air is pulled by the blood pump P4 in a direction reverse to the arrowed direction illustrated in FIG. 3 and mixed into the artery side blood line L3 by the amount of air included from the gap, and a volume (total amount of the dialysate and the included air) flowing into the hemodialyzer D also increases. Therefore, the fluid pressure of the dialysate flowing in the dialysate discharge line L2 does not decrease more than that in the normal state. This is verified by a real machine as indicated by the line F illustrated in FIG. 4.
Further, when an apparent connection failure that can be detected through visual inspection occurs in the connection portion between the hemodialyzer D and the artery side blood line L3, air flows into the hemodialyzer D and the artery side blood line L3 from the portion. That is, a large amount of air is pulled by the blood pump P4 in a direction reverse to the arrowed direction illustrated in FIG. 3 and flows into the artery side blood line L3, and a volume (total amount of the dialysate and the included air) flowing into the hemodialyzer D also remarkably increases. Therefore, the fluid pressure of the dialysate flowing in the dialysate discharge line L2 hardly varies. This is verified by a real machine as indicated by the line G illustrated in FIG. 4.

Further, the inventor of the present invention has verified, through a real machine, that the venous pressure measured by the venous pressure measuring means M_{V} disposed in the venous pressure monitor line L6 exhibits the same transition as that of the dialysate pressure measured by the dialysate pressure measuring means M_{T} if the circuit connection is normal in the case where the third fluid feeding means P3 is operated in the fluid removal direction, although not illustrated.

From the above-mentioned verification results, in the hemodialysis apparatus in which the normality of the circuit connection has been verified, a pressure variation amount in the dialysate pressure or the venous pressure when the clamp CL_{L5} is closed and the third fluid feeding means P3 is operated in the reverse filtration direction or the fluid removal direction, when a predetermined given period of time has elapsed since the start of priming, is measured and recorded under each of the priming conditions in advance, and set as the reference value in advance. As a result, in the hemodialysis apparatus whose normality is to be verified, a pressure variation amount in the dialysate pressure or the venous pressure during the above-mentioned operation is measured and compared with the reference value so as to judge the normality of the circuit connection. This is also one of the technical knowledge obtained by the inventor of the present invention.

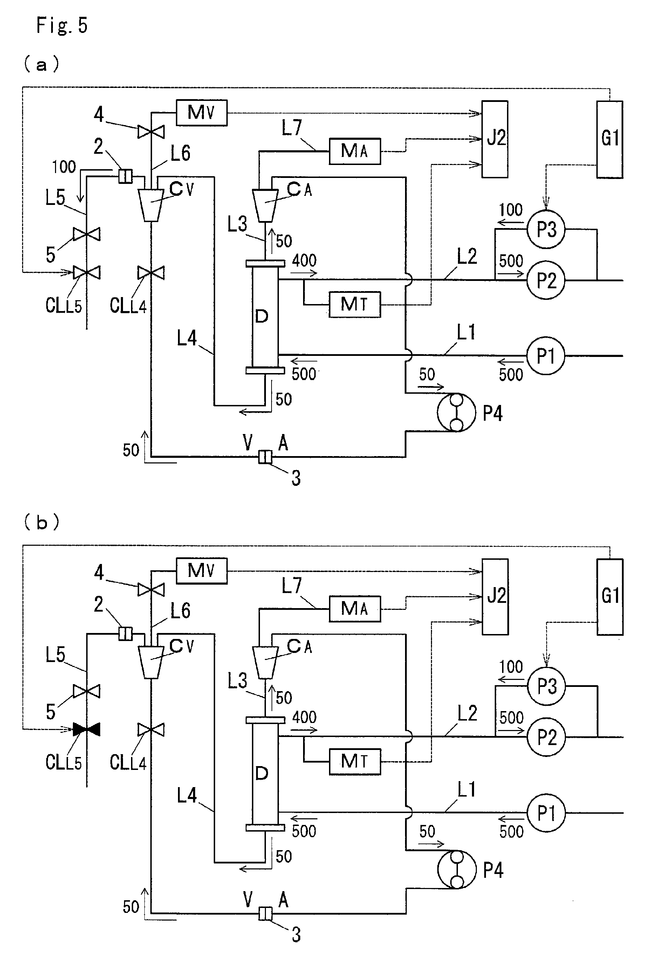

(D) The hemodialysis apparatus exemplified in FIGS. 1 and 3 includes a vein side chamber C_{V} in the vein side blood line L4, the dialysate pressure measuring means M_{T} for measuring the dialysate pressure which is a fluid pressure of the dialysate flowing in the dialysate discharge line L2 as the pressure measuring means, and the venous pressure measuring means M_{T} for measuring the venous pressure which is a pressure within the vein side blood line L4. The inventor of the present invention has verified, through a real machine, that the same result as the above-mentioned result is obtained in the hemodialysis apparatus of the mode illustrated in FIGS. 5, which includes the artery side chamber C_{A} in the artery side blood line L3, and arterial pressure measuring means M_{A} for measuring the arterial pressure which is a pressure within the artery side blood line L3 as the pressure measuring means.

That is, the inventor of the present invention has verified, through a real machine, that the venous pressure measured by the venous pressure measuring means M_{V} disposed in the venous pressure monitor line L6, and the arterial pressure measured by the arterial pressure measuring means M_{A} disposed in the arterial pressure monitor line L7 exhibit the same transition as that of the dialysate pressure measured by the dialysate pressure measuring means M_{T} if the circuit connection is normal when the above-mentioned operation is conducted, although not illustrated.

Accordingly, in the hemodialysis apparatus of the mode illustrated in FIGS. 5 inwhich the normality of the circuit connection has been verified, a pressure variation amount in any one of the dialysate pressure, the venous pressure, and the arterial pressure when the clamp CL_{L5} is closed and the third fluid feeding means P3 is operated in the reverse filtration direction or the fluid removal direction, when a predetermined given period of time has elapsed since the start of priming, is measured and recorded under each of the priming conditions in advance, and set as the reference value in advance. As a result, in the hemodialysis apparatus of the mode illustrated in FIGS. 5 whose normality is to be verified, a pressure variation amount in any one of the dialysate pressure, the venous pressure, and the arterial pressure during the above-mentioned operation is measured and compared with the reference value so as to judge the normality of the circuit connection. The above is the technical knowledge obtained by the inventor of the present invention with respect to the judgment of normality using the priming process.

(E) Next, plural technical findings obtained by the inventor of the present invention with respect to the judgment of normality using a blood removal process which is a subsequent process of the priming are described below.

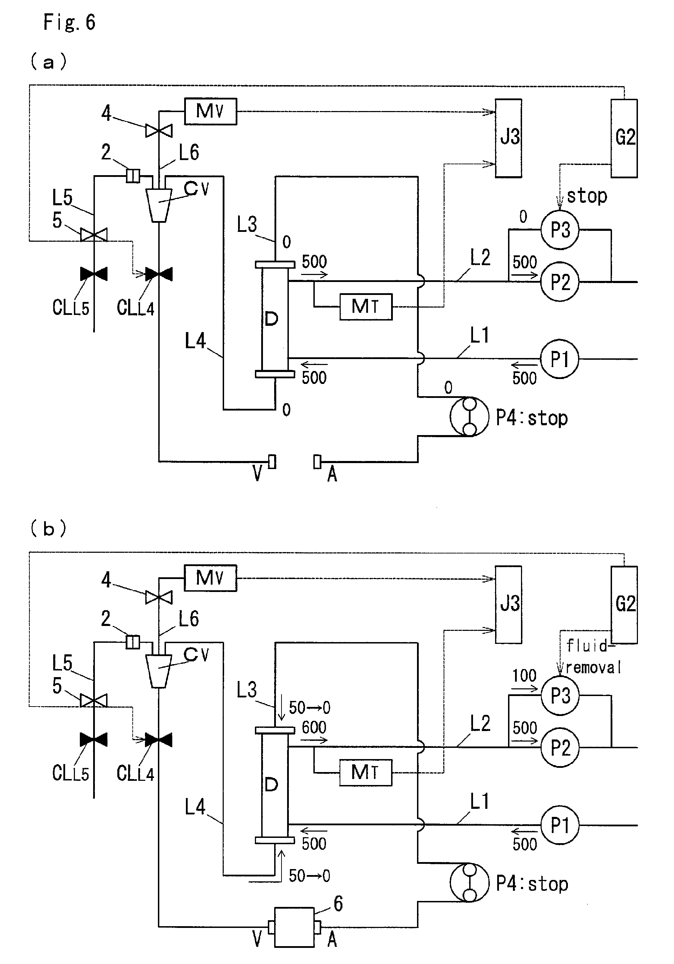

FIGS. 6 are diagrams illustrating a hemodialysis apparatus according to a third embodiment of the present invention, of which FIG. 6 (a) is a diagram illustrating a state in which the third fluid feeding means P3 stops for a predetermined period of time until blood removal starts, and FIG. 6(b) is a diagram illustrating an example of an operating state after the blood circuit is connected to an indwelling needle tapped into the patient to conduct the blood removal start operation.
It should be noted that numerical numbers illustrated in FIGS. 6 indicate flow rates of the dialysate flowing in the respective lines when the circuit connection is normal, and arrows indicate directions along which the dialysate flows.

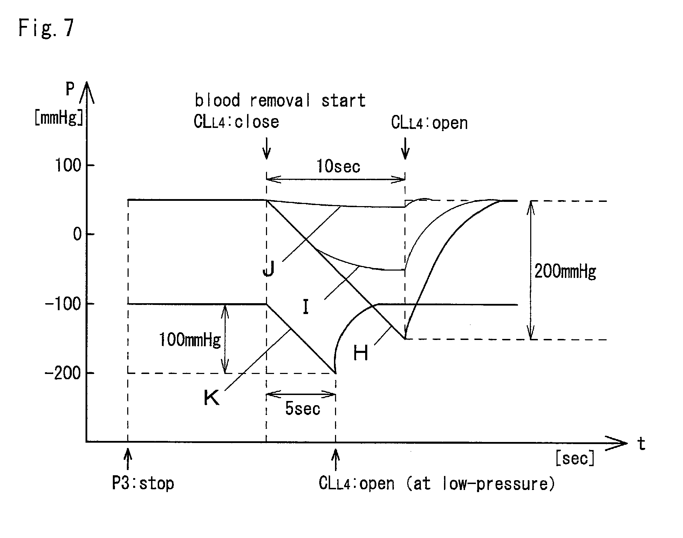

Further, FIG. 7 is a diagram schematically illustrating an example of a judgment principle using the blood removal process, and illustrates the transition of the dialysate pressure measured by the dialysate pressure measuring means M_{T} disposed in the dialysate discharge line L2.
A line H in the figure illustrates a transition of the dialysate pressure when the circuit connection is normal, a line I illustrates a transition of the dialysate pressure when a slight gap occurs in the connection portion between the hemodialyzer D and the artery side blood line L3, and a line J illustrates a transition of the dialysate pressure when an apparent connection failure that can be detected through visual inspection occurs in the connection portion between the hemodialyzer D and the artery side blood line L3.

As illustrated in FIGS. 7 and 6(b), a clamp CL_{L4} disposed on a downstream side of the vein side chamber C_{V} is closed, and the third fluid feeding means P3 is operated in the fluid removal direction when a predetermined given period of time has elapsed from the blood removal start operation.
With the above-mentioned operation, when the circuit connection is normal, the flow rate of the dialysate pulled from the hemodialyzer D is larger than the flow rate pushed into the hemodialyzer D, and the blood pump P4 stops. Therefore, the dialysate remaining in the artery side blood line L3 and the vein side blood line L4 flows into the hemodialyzer D, and the dialysate pressures in both of the artery side blood line L3 and the vein side blood line L4 decrease, and accordingly the fluid pressure of the dialysate flowing in the dialysate discharge line L2 decreases. This is verified by a real machine as indicated by the line H illustrated in FIG. 7. For example, it can be verified that the dialysate pressure decreases 200 mmHg within 10 sec when the clamp CL_{L4} is closed under the condition illustrated in FIG. 6(b).
FIG. 7 illustrates the transition of the dialysate pressure when the clamp CL_{L4} disposed on a downstream side of the vein side chamber C_{V} is closed, and the third fluid feeding means P3 is operated in the fluid removal direction together with the blood removal start operation as an example of the above-mentioned operation.

On the other hand, when a slight gap occurs in the connection portion between the hemodialyzer D and the artery side blood line L3, because air flows into the hemodialyzer D from the gap, the fluid pressure of the dialysate flowing in the dialysate discharge line L2 does not decrease more than that in the normal state. This is verified by a real machine as indicated by the line I illustrated in FIG. 7.
Further, when an apparent connection failure that can be detected through visual inspection occurs in the connection portion between the hemodialyzer D and the artery side blood line L3, a large amount of air flows into the hemodialyzer D from the portion. Therefore, the fluid pressure of the dialysate flowing in the dialysate discharge line L2 hardly varies. This is verified by a real machine as indicated by the line J illustrated in FIG. 7.

From the above-mentioned verification results, in the hemodialysis apparatus in which the normality of the circuit connection has been verified, a pressure variation amount in the dialysate pressure during the above-mentioned operation is measured and recorded under each of blood removal conditions in advance, and then set as a reference value in advance. As a result, in the hemodialysis apparatus whose normality is to be verified, a pressure variation amount in the dialysate pressure during the above-mentioned operation is measured and compared with the reference value so as to judge the normality of the circuit connection. This is one of the technical knowledge obtained by the inventor of the present invention.

(F) Further, the inventor of the present invention has verified, through a real machine, that the venous pressure measured by the venous pressure measuring means M_{V} disposed in the venous pressure monitor line L6 exhibits the same transition as that of the dialysate pressure measured by the dialysate pressure measuring means M_{T} if the circuit connection is normal when the above-mentioned operation is conducted, although not illustrated. This is also one of the technical knowledge obtained by the inventor of the present invention with respect to the judgment of normality using the blood removal process.
Accordingly, in the hemodialysis apparatus in which the normality of the circuit connection has been verified, a pressure variation amount in the dialysate pressure or the venous pressure when the clamp CL_{L4} is closed and the third fluid feeding means P3 is operated in the fluid removal direction, when a predetermined given period of time has elapsed from the blood removal start operation, is measured and recorded under each of the blood removal conditions in advance, and set as the reference value in advance. As a result, in the hemodialysis apparatus whose normality is to be verified, a pressure variation amount in the dialysate pressure or the venous pressure during the above-mentioned operation is measured and compared with the reference value so as to judge the normality of the circuit connection.

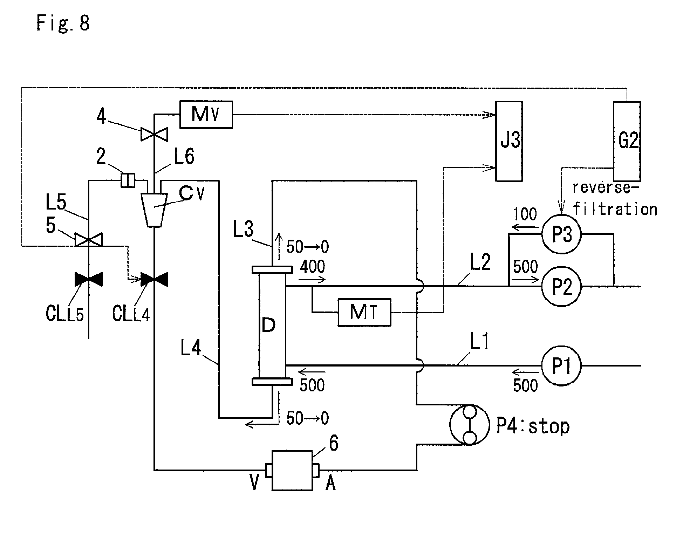

(G) It should be noted that the above-mentioned operation for judging the normality of the circuit connection described with reference to FIGS. 6 and 7 is to close the clamp CL_{L4} and operate the third feeding means P3 in the fluid removal direction. Alternatively, as illustrated in FIG. 8, the normality of the circuit connection can be judged by operating the third fluid feeding means P3 in the reverse filtration direction.

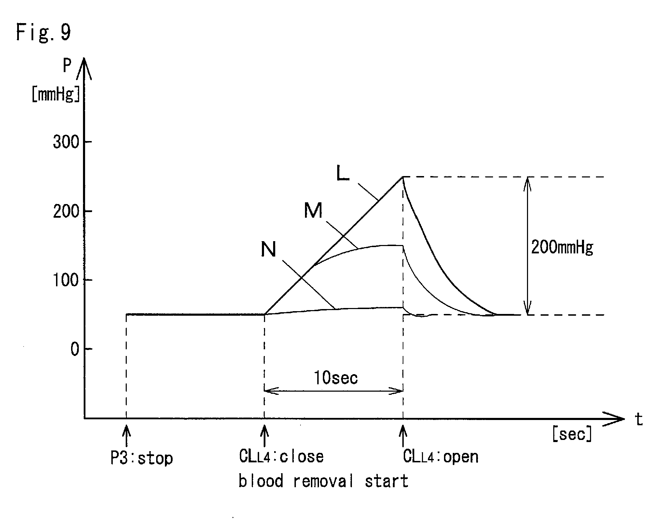

FIG. 9 is a diagram schematically illustrating an example of the judgment principle, and illustrates the transition of the dialysate pressure measured by the dialysate pressure measuring means M_{T} disposed in the dialysate discharge line L2.
A line L in the figure illustrates a transition of the dialysate pressure when the circuit connection is normal, a line M illustrates a transition of the dialysate pressure when a slight gap occurs in the connection portion between the hemodialyzer D and the artery side blood line L3, and a line N illustrates a transition of the dialysate pressure when an apparent connection failure that can be detected through visual inspection occurs in the connection portion between the hemodialyzer D and the artery side blood line L3.

As illustrated in FIGS. 9 and 8, the clamp CL_{L4} is closed, and the third fluid feeding means P3 is operated in the reverse filtration direction when a predetermined given period of time has elapsed from the blood removal start operation.
With the above-mentioned operation, when the circuit connection is normal, the dialysate that has been injected into both of the artery side blood line L3 and the vein side blood line L4 by reverse filtration has nowhere to go. Therefore, the dialysate pressures in both of the artery side blood line L3 and the vein side blood line L4 increase, and accordingly the fluid pressure of the dialysate flowing in the dialysate discharge line L2 also increases. This is verified by a real machine as indicated by the line L illustrated in FIG. 9. For example, it can be verified that the dialysate pressure increases 200 mmHg within 10 sec when the clamp CL_{L4} is closed under the condition illustrated in FIG. 8.
It should be noted that FIG. 9 illustrates the transition of the dialysate pressure when the clamp CL_{L4} disposed on the downstream side of the vein side chamber C_{V} is closed, and the third fluid feeding means P3 is operated in the reverse filtration direction together with the blood removal start operation as an example of the above-mentioned operation.

On the other hand, when a slight gap occurs in the connection portion between the hemodialyzer D and the artery side blood line L3, because the dialysate injected into both of the artery side blood line L3 and the vein side blood line L4 by reverse filtration is leaked from the gap, the fluid pressure of the dialysate flowing in the dialysate discharge line L2 does not increase more than that in the normal state. This is verified by a real machine as indicated by the line M illustrated in FIG. 9.
Further, when an apparent connection failure that can be detected through visual inspection occurs in the connection portion between the hemodialyzer D and the artery side blood line L3, a large amount of dialysate injected into both of the artery side blood line L3 and the vein side blood line L4 by reverse filtration is leaked from the gap. Therefore, the fluid pressure of the dialysate flowing in the dialysate discharge line L2 hardly varies. This is verified by a real machine as indicated by the line N illustrated in FIG. 9.

Further, the inventor of the present invention has verified, through a real machine, that the venous pressure measured by the venous pressure measuring means M_{V} disposed in the venous pressure monitor line L6 exhibits the same transition as that of the dialysate pressure measured by the dialysate pressure measuring means M_{T} if the circuit connection is normal also when the third fluid feedingmeans P3 is operated in the reverse filtration direction, although not illustrated.

From the above-mentioned verification results, in the hemodialysis apparatus in which the normality of the circuit connection has been verified, a pressure variation amount in the dialysate pressure or the venous pressure when the clamp CL_{L4} is closed and the third fluid feeding means P3 is operated in the fluid removal direction or the reverse filtration direction, when a predetermined given period of time has elapsed from the blood removal start operation, is measured and recorded under each of the blood removal conditions in advance, and set as the reference value in advance. As a result, in the hemodialysis apparatus whose normality is to be verified, a pressure variation amount in the dialysate pressure or the venous pressure during the above-mentioned operation is measured and compared with the reference value so as to judge the normality of the circuit connection. This is also one of the technical knowledge obtained by the inventor of the present invention.

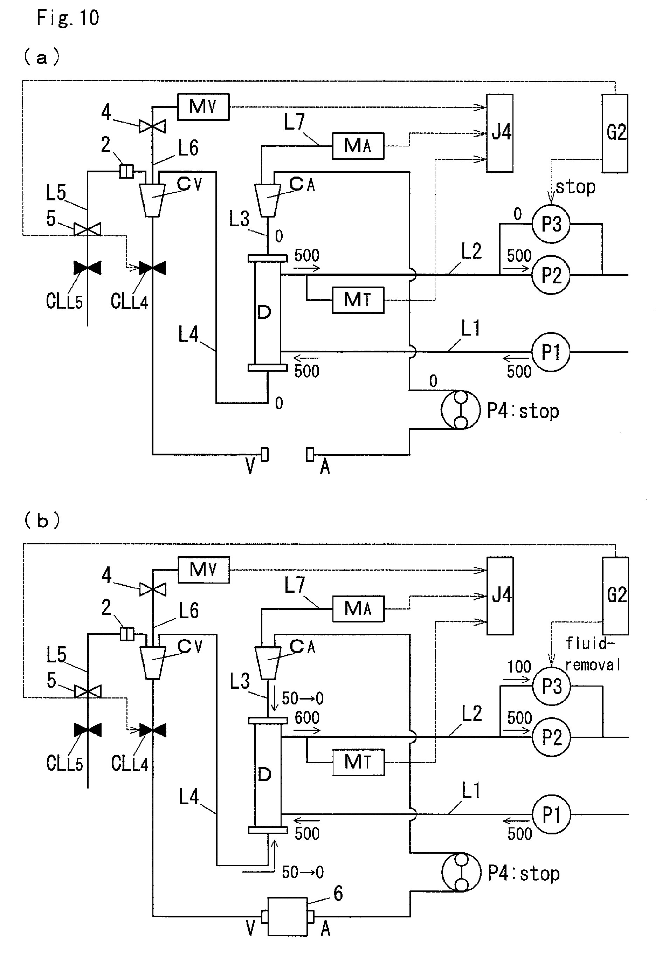

(H) In this example, the hemodialysis apparatus exemplified in FIGS. 6 and 8 includes the vein side chamber C_{V} in the vein side blood line L4, the dialysate pressure measuring means M_{T} for measuring the dialysate pressure which is a fluid pressure of the dialysate flowing in the dialysate discharge line L2 as the pressure measuring means, and the venous pressure measuring means M_{V} for measuring the venous pressure which is a pressure within the vein side blood line L4. However, the inventor of the present invention has verified, through a real machine, that the same result as the above-mentioned result is obtained in the hemodialysis apparatus of the mode illustrated in FIGS. 10, which includes the artery side chamber C_{A} in the artery side blood line L3, and the arterial pressure measuring means M_{A} for measuring the arterial pressure which is a pressure within the artery side blood line L3 as the pressure measuring means.

That is, the inventor of the present invention has verified, through a real machine, that the venous pressure measured by the venous pressure measuring means M_{V} disposed in the venous pressure monitor line L6, and the arterial pressure measured by the arterial pressure measuring means M_{A} disposed in the arterial pressure monitor line L7 exhibit the same transition as that of the dialysate pressure measured by the dialysate pressure measuring means M_{T} if the circuit connection is normal when the above-mentioned operation is conducted, although not illustrated.

Accordingly, in the hemodialysis apparatus of the mode illustrated in FIGS. 10 in which the normality of the circuit connection has been verified, a pressure variation amount in any one of the dialysate pressure, the venous pressure, and the arterial pressure when the clamp CL_{L4} is closed and the third fluid feeding means P3 is operated in the fluid removal direction or the reverse filtration direction, when a predetermined given period of time has elapsed from the blood removal start operation, is measured and recorded under each of the blood removal conditions in advance, and set as the reference value in advance. As a result, in the hemodialysis apparatus of the mode illustrated in FIGS. 10 whose normality is to be verified, a pressure variation amount in any one of the dialysate pressure, the venous pressure, and the arterial pressure during the above-mentioned operation is measured and compared with the reference value so as to judge the normality of the circuit connection. The above is the technical knowledge obtained by the inventor of the present invention with respect to the judgment of normality using the blood removal process.

Based on the above-mentioned knowledge, the inventor of the present invention has arrived at a hemodialysis apparatus that detects a minute connection failure that cannot be detected through visual inspection by the healthcare professional, which is represented by the so-called slow leak, so as to prevent beforehand any medical accident such as air inclusion or blood loss during the medical treatment. The summary is described below.

(1) A hemodialysis apparatus, including: a hemodialyzer D that brings blood into contact with a dialysate through a semipermeable membrane to purify the blood; a dialysate supply line L1 that supplies the dialysate to the hemodialyzer D; a dialysate discharge line L2 that discharges the dialysate from the hemodialyzer D; an artery side blood line L3 that allows the blood drawn from the patient to flow into the hemodialyzer D; a vein side blood line L4 that returns the blood drained from the hemodialyzer D to the patient; first fluid feeding means P1 disposed in the dialysate supply line L1; second fluid feeding means P2 disposed in the dialysate discharge line L2; reversible third fluid feeding means P3 disposed in a bypass line that communicates an upstream side and a downstream side of any one or both of the first fluid feeding means P1 and the second fluid feeding means P2 with each other; a blood pump P4 disposed in the artery side blood line L3; a vein side chamber C_{V} disposed on the vein side blood line L4; an overflow line L5 connected to the vein side chamber C_{V}; a venous pressure monitor line L6 connected to the vein side chamber C_{V}; dialysate pressure measuring means M_{T} that is disposed in the dialysate discharge line L2 and measures a dialysate pressure which is a fluid pressure of the dialysate flowing in the dialysate discharge line L2; venous pressure measuring means M_{V} that is connected to the venous pressure monitor line L6, and measures a venous pressure which is a pressure within the vein side blood line L4; a clamp CL_{L4} disposed on a downstream side of the vein side chamber C_{V}; a clamp CL_{L5} disposed in the overflow line L5; control means G1 that closes the clamp CL_{L5} when a predetermined given period of time has elapsed since the start of priming, and operates the third fluid feeding means P3 in a reverse filtration direction or a fluid removal direction, and then opens the clamp CL_{L5} when a predetermined given period of time has elapsed; and judging means J1 that compares a pressure variation amount in the dialysate pressure or the venous pressure for a period of time when the clamp CL_{L5} is closed with a predetermined reference value to judge normality of a circuit connection.

(2) A hemodialysis apparatus, including: hemodialyzer D that brings blood into contact with a dialysate through a semipermeable membrane to purify the blood; a dialysate supply line L1 that supplies the dialysate to the hemodialyzer D; a dialysate discharge line L2 that discharges the dialysate from the hemodialyzer D; an artery side blood line L3 that allows the blood drawn from the patient to flow into the hemodialyzer D; a vein side blood line L4 that returns the blood drained from the hemodialyzer D to the patient; first fluid feeding means P1 disposed in the dialysate supply line L1; second fluid feedingmeans P2 disposed in the dialysate discharge line L2; reversible third fluid feeding means P3 disposed in a bypass line that communicates an upstream side and a downstream side of any one or both of the first fluid feeding means P1 and the second fluid feeding means P2 with each other; a blood pump P4 disposed in the artery side blood line L3; a vein side chamber C_{V} disposed on the vein side blood line L4; an overflow line L5 connected to the vein side chamber C_{V}; a venous pressure monitor line L6 connected to the vein side chamber C_{V}; dialysate pressure measuring means M_{T} that is disposed in the dialysate discharge line L2 and measures a dialysate pressure which is a fluid pressure of the dialysate flowing in the dialysate discharge line L2; venous pressure measuring means M_{V} that is connected to the venous pressure monitor line L6, and measures a venous pressure which is a pressure within the vein side blood line L4; an artery side chamber C_{A} disposed on the artery side blood line L3; an arterial pressure monitor line L7 connected to the artery side chamber C_{A}; arterial pressure measuring means M_{A} that is connected to the arterial pressure monitor line L7, and measures an arterial pressure which is a pressure within the artery side blood line L3; a clamp CL_{L4} disposed on a downstream side of the vein side chamber C_{V}; a clamp CL_{L5} disposed in the overflow line L5; control means G1 that closes the clamp CL_{L5} when a predetermined given period of time has elapsed since the start of priming, and operates the third fluid feeding means P3 in a reverse filtration direction or a fluid removal direction, and then opens the clamp CL_{L5} when a predetermined given period of time has elapsed; and judging means J2 that compares a pressure variation amount in any one of the dialysate pressure, the venous pressure, and the arterial pressure for a period of time when the clamp CL_{L5} is closed with a predetermined reference value to judge normality of a circuit connection.

(3) The hemodialysis apparatus according to item (1), in which the control means G1 as described in item (1) is replaced with control means G2 that closes the clamp CL_{L4} when a predetermined given period of time has elapsed from a blood removal start operation, and operates the third fluid feeding means P3 in the fluid removal direction or the reverse filtration direction, and then opens the clamp CL_{L4} when a predetermined given period of time has elapsed, and the judging means J1 as described in item (1) is replaced with judging means J3 that compares a pressure variation amount in the dialysate pressure or the venous pressure for a period of time when the clamp CLL4 is closed with a predetermined reference value to judge normality of a circuit connection.

(4) The hemodialysis apparatus according to item (2), in which the control means G1 as described in item (2) is replaced with control means G2 that closes the clamp CL_{L4} when a predetermined given period of time has elapsed from a blood removal start operation, and operates the third fluid feeding means P3 in the fluid removal direction or the reverse filtration direction, and then opens the clamp CL_{L4} when a predetermined given period of time has elapsed, and the judging means J2 as described in item (2) is replaced with judging means J4 that compares a pressure variation amount in any one of the dialysate pressure, the venous pressure, and the arterial pressure for a period of time when the clamp CL_{L4} is closed with a predetermined reference value to judge normality of a circuit connection.

### Effects of the Invention

(1) The hemodialysis apparatus including the control means G1 and the judging means J1 exemplified in FIGS. 1 and 3 according to the present invention is a hemodialysis apparatus in which the normality of the circuit connection has been verified, in which a pressure variation amount in the dialysate pressure or the venous pressure when the clamp CL_{L5} is closed and the third fluid feeding means P3 is operated in the reverse filtration direction or the fluid removal direction, when a predetermined given period of time has elapsed since the start of priming, is measured and recorded under each of the priming conditions in advance, and set as the reference value in advance.
Accordingly, in the hemodialysis apparatus whose normality is to be verified, a pressure variation amount in the dialysate pressure or the venous pressure during the above-mentioned operation is measured and compared with the reference value so as to surely judge the normality of the circuit connection.

(2) The hemodialysis apparatus including the control means G1 and the judging means J2 exemplified in FIGS. 5 according to the present invention is a hemodialysis apparatus in which the normality of the circuit connection has been verified, in which a pressure variation amount in any one of the dialysate pressure, the venous pressure, and the arterial pressure when the clamp CL_{L5} is closed and the third fluid feeding means P3 is operated in the reverse filtration direction or the fluid removal direction, when a predetermined period of time has elapsed since the start of priming, is measured and recorded under each of the priming conditions in advance, and set as the reference value in advance.
Accordingly, in the hemodialysis apparatus whose normality is to be verified, a pressure variation amount in any one of the dialysate pressure, the venous pressure, and the arterial pressure during the above-mentioned operation is measured and compared with the reference value so as to surely judge the normality of the circuit connection.

(3) The hemodialysis apparatus including the control means G2 and the judging means J3 exemplified in FIGS. 6 and 8 according to the present invention is a hemodialysis apparatus in which the normality of the circuit connection has been verified, in which a pressure variation amount in the dialysate pressure or the venous pressure when the clamp CL_{L4} is closed and the third fluid feeding means P3 is operated in the fluid removal direction or the reverse filtration direction, when a predetermined given period of time has elapsed from the blood removal start operation, is measured and recorded under each of the blood removal conditions in advance, and set as the reference value in advance.
Accordingly, in the hemodialysis apparatus whose normality is to be verified, a pressure variation amount in the dialysate pressure or the venous pressure during the above-mentioned operation is measured and compared with the reference value so as to surely judge the normality of the circuit connection.

(4) The hemodialysis apparatus including the control means G2 and the judging means J4 exemplified in FIGS. 10 according to the present invention is a hemodialysis apparatus in which the normality of the circuit connection has been verified, in which a pressure variation amount in any one of the dialysate pressure, the venous pressure, and the arterial pressure when the clamp CL_{L4} is closed and the third fluid feeding means P3 is operated in the fluid removal direction or the reverse filtration direction, when a predetermined given period of time has elapsed from the blood removal start operation, is measured and recorded under each of the blood removal conditions in advance, and set as the reference value in advance.
Accordingly, in the hemodialysis apparatus whose normality is to be verified, a pressure variation amount in any one of the dialysate pressure, the venous pressure, and the arterial pressure during the above-mentioned operation is measured and compared with the reference value so as to surely judge the normality of the circuit connection.

(5) That is, the hemodialysis apparatus according to the above-mentioned modes (1) to (4) of the present invention can detect, in the preliminary phase for starting the medical treatment or in the imminent phase for starting the medical treatment, a minute connection failure that cannot be detected through visual inspection by the healthcare professional, which is represented by the so-called slow leak, so as to prevent beforehand any medical accident such as air inclusion or blood loss during the medical treatment. This contributes to provision of a safe dialysis treatment with no accident and a reduction in a physical strain and a mental strain on the healthcare professional, and the industrial availability thereof is extremely extensive.

### Brief Description of the Drawings

FIGS. 1 are diagrams illustrating a hemodialysis apparatus according to a first embodiment of the present invention, of which FIG. 1(a) is a diagram illustrating an operating state since the start of priming until a given period of time elapses, and FIG. 1(b) is a diagram illustrating an example of the subsequent operating state.
FIG. 2 is a diagram schematically illustrating an example of a judgment principle using a priming process, and a diagram illustrating a transition of a dialysate pressure measured by dialysate pressure measuring means M_{T}.
FIG. 3 is a diagram illustrating another example of the operating state after a given period of time has elapsed since the start of priming.
FIG. 4 is a diagram schematically illustrating another example of the judgment principle using the priming process, and a diagram illustrating the transition of the dialysate pressure measured by the dialysate pressure measuring means M_{T}.
FIGS. 5 are diagrams illustrating a hemodialysis apparatus according to a second embodiment of the present invention, of which FIG. 5(a) is a diagram illustrating an operating state since the start of priming until a given period of time elapses, and FIG. 5(b) is a diagram illustrating an example of the subsequent operating state.
FIGS. 6 are diagrams illustrating a hemodialysis apparatus according to a third embodiment of the present invention, of which FIG. 6(a) is a diagram illustrating a state in which third fluid feeding means P3 stops for a given period of time until blood removal starts, and FIG. 6(b) is a diagram illustrating an example of an operating state after a blood circuit is connected to an indwelling needle tapped into a patient to conduct a blood removal start operation.
FIG. 7 is a diagram schematically illustrating an example of a judgment principle using a blood removal process, and a diagram illustrating a transition of the dialysate pressure measured by the dialysate pressure measuring means M_{T}.
FIG. 8 is a diagram illustrating another example of an operating state after a blood circuit is connected to an indwelling needle tapped into a patient to conduct a blood removal start operation.
FIG. 9 is a diagram schematically illustrating another example of a judgment principle using the blood removal process, and a diagram illustrating the transition of the dialysate pressure measured by the dialysate pressure measuring means M_{T}.
FIGS. 10 are diagrams illustrating a hemodialysis apparatus according to a fourth embodiment of the present invention, of which FIG. 10(a) is a diagram illustrating a state in which third fluid feeding means P3 stops for a given period of time until blood removal starts, and FIG. 10(b) is a diagram illustrating an example of an operating state after a blood circuit is connected to an indwelling needle tapped into a patient to conduct a blood removal start operation.

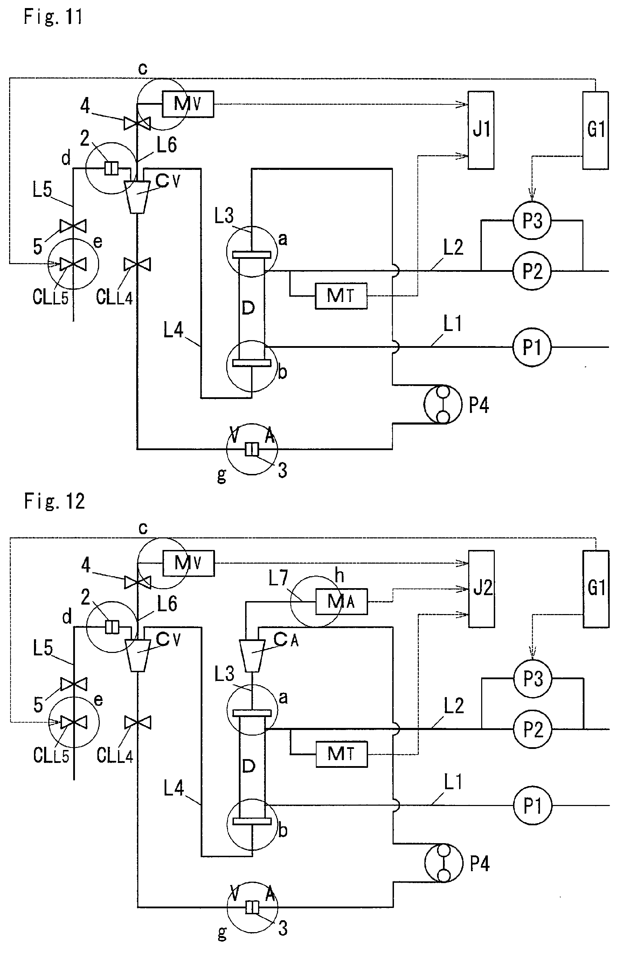

FIG. 11 is a diagram illustrating connection portions whose normality can be verified according to the first embodiment.
FIG. 12 is a diagram illustrating connection portions whose normality can be verified according to the second embodiment.

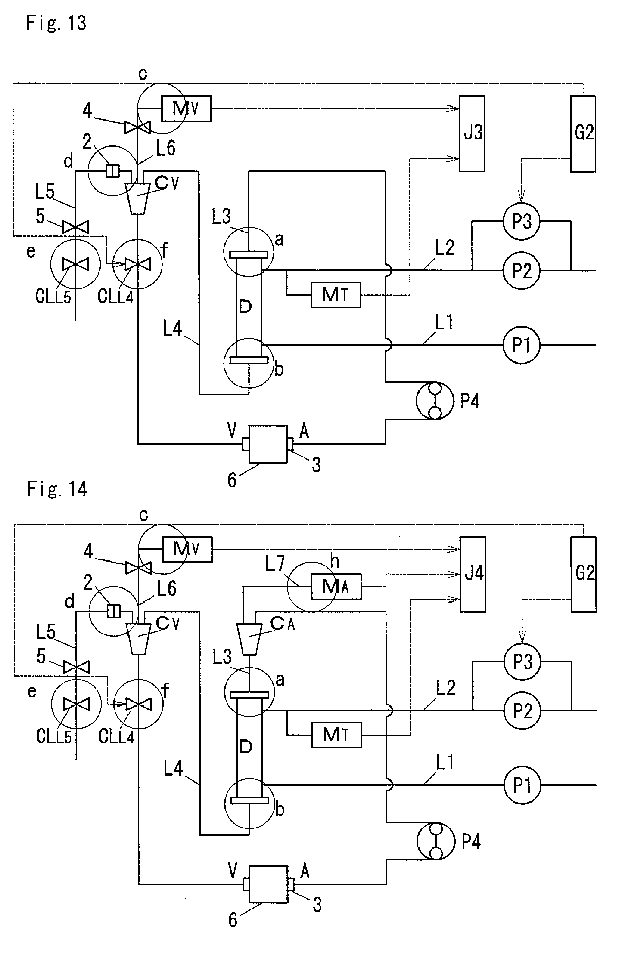

FIG. 13 is a diagram illustrating connection portions whose normality can be verified according to the third embodiment.
FIG. 14 is a diagram illustrating connection portions whose normality can be verified according to the fourth embodiment.

### Description of Symbols

- 1:: hemodialysis apparatus

- 2:: junction joint
- 3:: bypass joint
- 4:: forceps
- 5:: forceps
- 6:: patient
- C_{A}:: artery side chamber
- C_{V}:: vein side chamber
- CL_{L4}:: clamp disposed on downstream side of vein side chamber C_{V}
- CL_{L5}:: clamp disposed in overflow line L5
- D:: hemodialyzer
- G1,: G2: control means
- J1,: J2, J3, J4: judging means
- L1:: dialysate supply line
- L2:: dialysate discharge line
- L3:: artery side blood line
- L4:: vein side blood line
- L5:: overflow line
- L6:: venous pressure monitor line
- L7:: arterial pressure monitor line
- M_{A}:: arterial pressure measuring means
- M_{T}:: dialysate pressure measuring means
- M_{V}:: venous pressure measuring means
- P1:: first fluid feeding means
- P2:: second fluid feeding means
- P3:: third fluid feeding means
- P4:: blood pump

### Best modes for carrying out the Invention

Best modes for carrying out the present invention are described below with reference to FIGS. 1 to 14.
FIGS. 1 and 3 are diagrams illustrating a hemodialysis apparatus according to a first embodiment of the present invention.
The hemodialysis apparatus of the first embodiment is configured to judge normality of a circuit connection by using a priming process, and includes dialysate pressure measuring means M_{T}, venous pressure measuring means M_{V}, control means G1, and judging means J1 as illustrated in FIGS. 1 and 3.
The dialysate pressure measuring means M_{T} disposed in a dialysate discharge line L2 measures a dialysate pressure which is a fluid pressure of a dialysate flowing in the dialysate discharge line L2, and the venous pressure measuring means M_{V} disposed in a venous pressure monitor line L6 measures a venous pressure which is a pressure within a vein side blood line L4.

The control means G1 closes a clamp CL_{L5} when a predetermined given period of time has elapsed since the start of priming, and operates third fluid feeding means P3 in a reverse filtration direction or a fluid removal direction, and then opens the clamp CL_{L5} when a predetermined given period of time has elapsed, based on information recorded in given recording means. FIG. 1(b) is a state diagram when the third fluid feeding means P3 is operated in the reverse filtration direction, and FIG. 3 is a state diagram when the third fluid feeding means P3 is operated in the fluid removal direction.
Then, the judging means J1 compares a pressure variation amount in a dialysate pressure or a venous pressure for a period of time when the clamp CL_{L5} is closed with a predetermined reference value to judge the normality of the circuit connection.

In this example, the predetermined reference value is a measured pressure variation amount which has been recorded in given recording means such as a memory or a hard disk in advance after, in the hemodialysis apparatus in which the normality of the circuit connection has been verified, a pressure variation amount in the dialysate pressure or the venous pressure when the clamp CL_{L5} is closed and the third fluid feeding means P3 is operated in the reverse filtration direction or the fluid removal direction, when the predetermined given period of time has elapsed since the start of priming, is measured under each of the priming conditions in advance.

For example, when the third fluid feeding means P3 is operated in the reverse filtration direction as illustrated in FIG. 1(b), in the case where the circuit connection is normal, the dialysate that has been discharged from an overflow line L5 during priming has nowhere to go, and the dialysate pressures in both of an artery side blood line L3 and the vein side blood line L4 increase, and accordingly the fluid pressure of the dialysate flowing in the dialysate discharge line L2 also increases. Under a condition illustrated in FIG. 1(b), the dialysate pressure increases 200 mmHg within 5 sec when the clamp CL_{L5} is closed as indicated by a line A illustrated in FIG. 2.
Further, because the venous pressure also passes through a semipermeable membrane of a hemodialyzer D, under the condition illustrated in FIG. 1(b), although not illustrated, the venous pressure increases 200 mmHg within 5 sec when the clamp CL_{L5} is closed, similarly to the line A illustrated in FIG. 2.

On the other hand, when the third fluid feeding means P3 is operated in the fluid removal direction as illustrated in FIG. 3, in the case where the circuit connection is normal, the flow rate of the dialysate pushed into the hemodialyzer D is smaller than the pulled flow rate. Therefore, the dialysate pressures in both of the artery side blood line L3 and the vein side blood line L4 decrease, and accordingly the fluid pressure of the dialysate flowing in the dialysate discharge line L2 also decreases. Under the condition illustrated in FIG. 3, the dialysate pressure decreases 200 mmHg within 5 sec when the clamp CL_{L5} is closed as indicated by a line E illustrated in FIG. 4.
Further, because the venous pressure also passes through the semipermeable membrane of the hemodialyzer D, under the condition illustrated in FIG. 3, although not illustrated, the venous pressure decreases 200 mmHg within 5 sec when the clamp CL_{L5} is closed, similarly to the line E illustrated in FIG. 4.
As described above, the pressure variation amount in the dialysate pressure or the venous pressure which has been measured under each of the priming conditions in advance, and recorded in the given recording means such as a memory or a hard disk in advance is the predetermined reference value.

Then, according to the first embodiment, in the hemodialysis apparatus whose normality is to be verified, a pressure variation amount in the dialysate pressure or the venous pressure during the above-mentioned operation is measured and compared with the reference value so as to judge the normality of the circuit connection.

The period of time until the clamp CL_{L5} is closed since the start of priming is not particularly restricted, but it is desirable that the clamp CL_{L5} be closed after the dialysate that has washed the artery side blood line L3 and the dialysate that has washed the vein side blood line L4 have been discharged from the overflow line L5. In other words, it is desirable that the clamp CL_{L5} be closed after the artery side blood line L3 and the vein side blood line L4 have been filled with the dialysate.
Accordingly, it is desirable that, in the hemodialysis apparatus in which the normality of the circuit connection has been verified, a period of time since the start of priming until the artery side blood line L3 and the vein side blood line L4 are filled with the dialysate be measured, and the period of time be recorded in the given recording means such as a memory or a hard disk as a predetermined period of time since the start of priming until the clamp CL_{L5} is closed.

A period of time during which the clamp CL_{L5} is closed is not particularly restricted. A positive pressure is applied to the hemodialyzer D as illustrated in FIG. 2 when the third fluid feeding means P3 is operated in the reverse filtration direction, and a negative pressure is applied to the hemodialyzer D as illustrated in FIG. 4 when the third fluid feeding means P3 is operated in the fluid removal direction. Therefore, it is desirable to implement the closing of the clamp CL_{L5} in a range where the hemodialyzer D does not induce withstand pressure breakdown.

For example, the line A illustrated in FIG. 2 illustrates the transition of the dialysate pressure when the circuit connection is normal. Because the withstand pressure of the hemodialyzer D used in this case is 500 mmHg (generally about 500 mmHg in many cases), a positive pressure is applied for 5 seconds during which the dialysate pressure increases from 100 mmHg to 300 mmHg with a margin.
Further, a line D illustrated in FIG. 2 also illustrates the transition of the dialysate pressure when the circuit connection is normal. However, because the hemodialyzer D used in this case is low in permeability, the dialysate pressure has already reached 250 mmHg before the clamp CL_{L5} is closed. In this state, when a positive pressure is applied for 5 seconds as with the line A, there is a risk that the hemodialyzer D induces the withstand pressure breakdown. Accordingly, in this case, a positive pressure is applied for 2.5 seconds during which the dialysate pressure increases from 250 mmHg to 350 mmHg.

As described above, the dialysate pressure before the clamp CL_{L5} is closed, and the pressure variation amount in the dialysate pressure during a period of time when the clamp CL_{L5} is closed varies according to the hemodialyzer D to be used, particularly the permeability thereof. Therefore, it is desirable that, in the hemodialysis apparatus in which the normality of the circuit connection using the hemodialyzer D has been verified, a period of time when the dialysate pressure or the venous pressure increases, for example, from 100 mmHg to 300 mmHg be measured, and the measured period of time be recorded in the given recording means such as a memory or a hard disk as a predetermined given period of time since the clamp CL_{L5} is closed and until the clamp CL_{L5} is opened.

A case where the circuit connection is normal in the first embodiment means a case where all portions indicated below are normal, and those portions are illustrated in FIG. 11. In other words, according to the first embodiment, in the hemodialysis apparatus whose normality is to be verified, a pressure variation amount in the dialysate pressure or the venous pressure during the above-mentioned operation is measured and compared with the reference value so as to surely judge the normality of the following portions.
a: The hemodialyzer D and the artery side blood line L3 are normally connected to each other.
b: The hemodialyzer D and the vein side blood line L4 are normally connected to each other.
c: The venous pressure measuring means M_{V} is normally connected.
d: A junction joint 2 is normally connected.
e: The clamp CL_{L5} is normally loaded.
g: A bypass joint 3 is normally connected.

FIGS. 5 are diagrams illustrating a hemodialysis apparatus according to a second embodiment of the present invention.
The hemodialysis apparatus of the second embodiment is also configured to judge the normality of the circuit connection by using the priming process as in the first embodiment. As illustrated in FIGS. 5, the hemodialysis apparatus includes the dialysate pressure measuring means M_{T}, the venous pressure measuring means M_{V}, the arterial pressure measuring means M_{A}, the control means G1, and judging means J2. The dialysate pressure measuring means M_{T}, the venous pressure measuring means M_{V}, and the control means G1 are identical with those in the first embodiment.
The arterial pressure measuring means M_{A} disposed in the arterial pressure monitor line L7 measures the arterial pressure which is a pressure within the artery side blood line L3.

The control means G1 closes the clamp CL_{L5} when a predetermined given period of time has elapsed since the start of priming, and operates the third fluid feeding means P3 in the reverse filtration direction or the fluid removal direction based on information recorded in the given recording means as in the first embodiment, and then opens the clamp CL_{L5} when the predetermined given period of time has elapsed. FIG. 5(a) is a diagram illustrating an operating state since the start of priming until a given period of time elapses, and FIG. 5(b) is a diagram illustrating an example of a subsequent operating state, which is a state diagram when the third fluid feeding means P3 is operated in the reverse filtration direction.
The judging means J2 compares a pressure variation amount in any one of the dialysate pressure, the venous pressure, and the arterial pressure for a period of time when the clamp CL_{L5} is closed, with the predetermined reference value to judge the normality of the circuit connection.

The predetermined reference value is a measured pressure variation amount which has been recorded in the given recording means such as a memory or a hard disk in advance after, in the hemodialysis apparatus in which the normality of the circuit connection has been verified, a pressure variation amount in any one of the dialysate pressure, the venous pressure, and the arterial pressure when the clamp CL_{L5} is closed and the third fluid feeding means P3 is operated in the reverse filtration direction or the fluid removal direction, when the predetermined given period of time has elapsed since the start of priming, is measured under each of the priming conditions in advance.

For example, when the third fluid feeding means P3 is operated in the reverse filtration direction as illustrated in FIG. 5(b), in the case where the circuit connection is normal, the dialysate that has been discharged from the overflow line L5 during priming has nowhere to go, and the dialysate pressures in both of the artery side blood line L3 and the vein side blood line L4 increase, and accordingly the fluid pressure of the dialysate flowing in the dialysate discharge line L2 also increases. Under a condition illustrated in FIG. 5(b), although not illustrated, the dialysate pressure increases 200 mmHg within 5 sec when the clamp CL_{L5} is closed, similarly to the line A illustrated in FIG. 2.
Further, because the venous pressure and the arterial pressure pass through the semipermeable membrane of the hemodialyzer D, under the condition illustrated in FIG. 5(b), although not illustrated, the venous pressure and the arterial pressure increase 200 mmHg within 5 sec when the clamp CL_{L5} is closed, similarly to the line A illustrated in FIG. 2.

On the other hand, although not illustrated, when the thirdfluidfeeding means P3 is operated in the fluid removal direction, in the case where the circuit connection is normal, the flow rate of the dialysate pushed into the hemodialyzer D is smaller than the pulled flow rate. Therefore, the dialysate pressures in both of the artery side blood line L3 and the vein side blood line L4 decrease, and accordingly the fluid pressure of the dialysate flowing in the dialysate discharge line L2 also decreases. Under this condition, although not illustrated, the dialysate pressure decreases 200 mmHg within 5 sec when the clamp CL_{L5} is closed, similarly to the line E illustrated in FIG. 4.
Further, because the venous pressure and the arterial pressure pass through the semipermeable membrane of the hemodialyzer D, under this condition, although not illustrated, the venous pressure and the arterial pressure decrease 200 mmHg within 5 sec when the clamp CL_{L5} is closed, similarly to the line E illustrated in FIG. 4.
As described above, the pressure variation amount in any one of the dialysate pressure, the venous pressure, and the arterial pressure, which has been measured under each of the priming conditions in advance, and recorded in the given recording means such as a memory or a hard disk in advance is the predetermined reference value.

Then, according to the second embodiment, in the hemodialysis apparatus whose normality is to be verified, a pressure variation amount in any one of the dialysate pressure, the venous pressure, and the arterial pressure during the above-mentioned operation is measured and compared with the reference value so as to judge the normality of the circuit connection.

The period of time until the clamp CL_{L5} is closed since the start of priming, and a period of time during which the clamp CL_{L5} is closed are identical with those in the first embodiment.

A case where the circuit connection is normal in the second embodiment means a case where all portions indicated below are normal, and those portions are illustrated in FIG. 12. In other words, according to the second embodiment, in the hemodialysis apparatus whose normality is to be verified, a pressure variation amount in any one of the dialysate pressure, the venous pressure, and the arterial pressure during the above-mentioned operation is measured and compared with the reference value so as to surely judge the normality of the following portions.
a: The hemodialyzer D and the artery side blood line L3 are normally connected to each other.
b: The hemodialyzer D and the vein side blood line L4 are normally connected to each other.
c: The venous pressure measuring means M_{V} is normally connected.
d: The junction joint 2 is normally connected.
e: The clamp CL_{L5} is normally loaded.
g: The bypass joint 3 is normally connected.
h: The arterial pressure measuring means M_{A} is normally connected.

FIGS. 6 and 8 are diagrams illustrating a hemodialysis apparatus according to a third embodiment of the present invention.
The hemodialysis apparatus of the third embodiment is configured to judge the normality of the circuit connection by using a blood removal process. As illustrated in FIGS. 6 and 8, the hemodialysis apparatus includes the dialysate pressure measuring means M_{T}, the venous pressure measuring means M_{V}, control means G2, and judging means J3. The dialysate pressure measuring means M_{T} and the venous pressure measuring means M_{V} are identical with those in the first embodiment.

The control means G2 closes a clamp CL_{L4} when a predetermined given period of time has elapsed from a blood removal start operation, and operates the third fluid feeding means P3 in the fluid removal direction or the reverse filtration direction based on information recorded in given recording means, and then opens the clamp CL_{L4} when a predetermined given period of time has elapsed. FIG. 6(a) is a diagram illustrating a state in which the third fluid feeding means P3 stops for a given period of time until blood removal starts, FIG. 6(b) is a diagram illustrating an example of an operating state after a blood circuit is connected to an indwelling needle tapped into the patient to conduct the blood removal start operation, and a state diagram when the third fluid feeding means P3 is operated in the fluid removal direction, and FIG. 8 is a state diagram when the third fluid feeding means P3 is operated in the reverse filtration direction.
Then, the judging means J3 compares a pressure variation amount in the dialysate pressure or the venous pressure for a period of time when the clamp CL_{L4} is closed, with the predetermined reference value to judge the normality of the circuit connection.

In this example, the predetermined reference value is a measured pressure variation amount which has been recorded in the given recording means such as a memory or a hard disk in advance after, in the hemodialysis apparatus in which the normality of the circuit connection has been verified, a pressure variation amount in the dialysate pressure or the venous pressure when the clamp CL_{L4} is closed and the third fluid feeding means P3 is operated in the fluid removal direction or the reverse filtration direction, when the predetermined given period of time has elapsed from the blood removal start operation, is measured under each of blood removal conditions in advance.

For example, when the third fluid feeding means P3 is operated in the fluid removal direction as illustrated in FIG. 6(b), in the case where the circuit connection is normal, the flow rate of the dialysate pulled from the hemodialyzer D is larger than the flow rate pushed into the hemodialyzer D, and a blood pump P4 stops. Therefore, the dialysate remaining in the artery side blood line L3 and the vein side blood line L4 flows into the hemodialyzer D, and the dialysate pressures in both of the artery side blood line L3 and the vein side blood line L4 decrease, and accordingly the fluid pressure of the dialysate flowing in the dialysate discharge line L2 decreases. Under the condition illustrated in FIG. 6(b), the dialysate pressure decreases 200 mmHg within 10 sec when the clamp CL_{L4} is closed as indicated by a line H illustrated in FIG. 7.
Further, because the venous pressure passes through the semipermeable membrane of the hemodialyzer D, under the condition illustrated in FIG. 6(b), although not illustrated, the venous pressure decreases 200 mmHg within 10 sec when the clamp CL_{L4} is closed, similarly to the line H illustrated in FIG. 7.

On the other hand, when the third fluid feeding means P3 is operated in the reverse filtration direction as illustrated in FIG. 8, in the case where the circuit connection is normal, the dialysate that has been injected into both of the artery side blood line L3 and the vein side blood line L4 by reverse filtration has nowhere to go. Therefore, the dialysate pressures in both of the artery side blood line L3 and the vein side blood line L4 increase, and accordingly the fluid pressure of the dialysate flowing in the dialysate discharge line L2 also increases. Under the condition illustrated in FIG. 8, the dialysate pressure increases 200 mmHg within 10 sec when the clamp CL_{L4} is closed as indicated by a line L illustrated in FIG. 9.
Further, becasue the venous pressure also passes through the semipermeable membrane of the hemodialyzer D, under the condition illustrated in FIG. 8, although not illustrated, the venous pressure increases 200 mmHg within 10 sec when the clamp CL_{L4} is closed, similarly to the line L illustrated in FIG. 9.
As described above, the pressure variation amount in the dialysate pressure or the venous pressure which has been measured under each of the blood removal conditions in advance, and recorded in the given recording means such as a memory or a hard disk in advance is the predetermined reference value.

Then, according to the third embodiment, in the hemodialysis apparatus whose normality is to be verified, a pressure variation amount in the dialysate pressure or the venous pressure during the above-mentioned operation is measured and compared with the reference value so as to judge the normality of the circuit connection.

The period of time from the blood removal start operation until the clamp CL_{L4} is closed is not particularly restricted. However, from the viewpoint of reducing a strain on the patient into which the indwelling needle connected to the blood circuit is tapped, it is desirable to close the clamp CL_{L4} together with the blood removal start operation.
The blood removal start operation is an operation in which after the blood circuit is connected to the indwelling needle tapped into the patient, the healthcare professional who manipulates the hemodialysis apparatus verifies that blood removal start is prepared, and gives instructions for operating the third fluid feeding means P3 in the fluid removal direction and operating the blood pump P4 in the forward rotation direction to the hemodialysis apparatus. For example, the healthcare professional presses a blood removal start button disposed in the hemodialysis apparatus so that the third f luid feedingmeans P3 is operated in the fluid removal direction, and the blood pump P4 is operated in the forward rotation direction, so as to start blood removal from the patient. The forward rotation direction is a direction of rotating the blood pump during hemodialysis treatment, and the reverse rotation direction is a direction reverse to the forward rotation direction.

A period of time during which the clamp CL_{L4} is closed is not particularly restricted. A negative pressure is applied to the hemodialyzer D as illustrated in FIG. 7 when the third fluid feeding means P3 is operated in the fluid removal direction, and a positive pressure is applied to the hemodialyzer D as illustrated in FIG. 9 when the third fluid feeding means P3 is operated in the reverse filtration direction. Therefore, it is desirable to implement the closing of the clamp CL_{L4} in a range where the hemodialyzer D does not induce withstand pressure breakdown.

For example, the line H illustrated in FIG. 7 illustrates the transition of the dialysate pressure when the circuit connection is normal. Because the withstand pressure of the hemodialyzer D used in this case is 500 mmHg, a negative pressure is applied for 10 seconds during which the dialysate pressure decreases from 50 mmHg to -150 mmHg with a margin. Further, a line K illustrated in FIG. 7 also illustrates the transition of the dialysate pressure when the circuit connection is normal. However, because the hemodialyzer D used in this case is low in permeability, the dialysate pressure has already reached -100 mmHg before the clamp CL_{L4} is closed. In this state, when a negative pressure is applied for 10 seconds as with the line H, there is a risk that the hemodialyzer D induces the withstand pressure breakdown. Accordingly, in this case, a negative pressure is applied for 5 seconds until the dialysate pressure decreases from -100 mmHg to -200 mmHg.

As described above, the dialysate pressure before the clamp CL_{L4} is closed, and the pressure variation amount in the dialysate pressure during a period of time when the clamp CL_{L4} is closed varies according to the hemodialyzer D to be used, particularly the permeability thereof. Therefore, it is desirable that, in the hemodialysis apparatus in which the normality of the circuit connection using the hemodialyzer D has been verified, a period of time when the dialysate pressure or the venous pressure decreases, for example, from 50 mmHg to -150 mmHg be measured, and the measured period of time be recorded in the given recording means such as a memory or a hard disk as a predetermined given period of time since the clamp CL_{L4} is closed and until the clamp CL_{L4} is opened.

A case where the circuit connection is normal in the third embodiment means a case where all portions indicated below are normal, and those portions are illustrated in FIG. 13. In other words, according to the third embodiment, in the hemodialysis apparatus whose normality is to be verified, a pressure variation amount in the dialysate pressure or the venous pressure during the above-mentioned operation is measured and compared with the reference value so as to surely judge the normality of the following portions.
a: The hemodialyzer D and the artery side blood line L3 are normally connected to each other.
b: The hemodialyzer D and the vein side blood line L4 are normally connected to each other.
c: The venous pressure measuring means M_{V} is normally connected.
d: The junction joint 2 is normally connected.
e: The clamp CL_{L5} is normally loaded.
f: The clamp CL_{L4} is normally loaded.

FIGS. 10 are diagrams illustrating a hemodialysis apparatus according to a fourth embodiment of the present invention.
The hemodialysis apparatus of the fourth embodiment is also configured to judge the normality of the circuit connection by using the blood removal process as in the third embodiment. As illustrated in FIGS. 10, the hemodialysis apparatus includes the dialysate pressure measuring means M_{T}, the venous pressure measuring means M_{V}, the arterial pressure measuring means M_{A}, the control means G2, and judging means J4. The dialysate pressure measuring means M_{T}, the venous pressure measuring means M_{V}, and the control means G2 are identical with those in the third embodiment. Further, the arterial pressure measuring means M_{A} is identical with that in the second embodiment.

Similarly to the third embodiment, the control means G2 closes the clamp CL_{L4} when a predetermined given period of time has elapsed from the blood removal start operation, and operates the third fluid feeding means P3 in the fluid removal direction or the reverse filtration direction based on information recorded in the given recording means, and then opens the clamp CL_{L4} when a predetermined given period of time has elapsed. FIG. 10(a) is a diagram illustrating a state in which the third fluid feeding means P3 stops for a given period of time until blood removal starts, FIG. 10(b) is a diagram illustrating an example of an operating state after the blood circuit is connected to an indwelling needle tapped into the patient to conduct the blood removal start operation, and a state diagram when the third fluid feeding means P3 is operated in the fluid removal direction.
The judging means J4 compares a pressure variation amount in any one of the dialysate pressure, the venous pressure, and the arterial pressure for a period of time when the clamp CL_{L4} is closed, with the predetermined reference value to judge the normality of the circuit connection.

The predetermined reference value is a measured pressure variation amount which has been recorded in the given recording means such as a memory or a hard disk in advance after, in the hemodialysis apparatus in which the normality of the circuit connection has been verified, a pressure variation amount in any one of the dialysate pressure, the venous pressure, and the arterial pressure when the clamp CL_{L4} is closed and the third fluid feeding means P3 is operated in the fluid removal direction or the reverse filtration direction, when the predetermined given period of time has elapsed from the blood removal start operation, is measured under each of the blood removal conditions in advance.

For example, when the third fluid feeding means P3 is operated in the fluid removal direction as illustrated in FIG. 10(b), in the case where the circuit connection is normal, the flow rate of the dialysate pulled from the hemodialyzer D is larger than the flow rate pushed into the hemodialyzer D, and the blood pump P4 stops. Therefore, the dialysate remaining in the artery side blood line L3 and the vein side blood line L4 flows into the hemodialyzer D, and the dialysate pressures in both of the artery side blood line L3 and the vein side blood line L4 decrease, and accordingly the fluid pressure of the dialysate flowing in the dialysate discharge line L2 decreases. Under the condition illustrated in FIG. 10(b), although not illustrated, the dialysate pressure decreases 200 mmHg within 10 sec when the clamp CL_{L4} is closed, similarly to the line H illustrated in FIG 7.
Further, because the venous pressure and the arterial pressure pass through the semipermeable membrane of the hemodialyzer D, under the condition illustrated in FIG. 10(b), although not illustrated, the venous pressure and the arterial pressure decrease 200 mmHg within 10 sec when the clamp CL_{L4} is closed, similarly to the line H illustrated in FIG. 7.

On the other hand, although not illustrated, when the third fluid feeding means P3 is operated in the reverse filtration direction, in the case where the circuit connection is normal, the dialysate that has been injected into both of the artery side blood line L3 and the vein side blood line L4 by reverse filtration has nowhere to go. Therefore, the dialysate pressures in both of the artery side blood line L3 and the vein side blood line L4 increase, and accordingly the fluid pressure of the dialysate flowing in the dialysate discharge line L2 also increases. Under this condition, although not illustrated, the dialysate pressure increases 200 mmHg within 10 sec when the clamp CL_{L4} is closed, similarly to the line L illustrated in FIG. 9.
Further, because the venous pressure and the arterial pressure pass through the semipermeable membrane of the hemodialyzer D, under this condition, although not illustrated, the venous pressure and the arterial pressure increase 200 mmHg within 10 sec when the clamp CL_{L4} is closed, similarly to the line L illustrated in FIG. 9.
As described above, the pressure variation amount in any one of the dialysate pressure, the venous pressure, and the arterial pressure, which has been measured under each of the blood removal conditions in advance, and recorded in the given recording means such as a memory or a hard disk in advance is the predetermined reference value.

Then, according to the fourth embodiment, in the hemodialysis apparatus whose normality is to be verified, a pressure variation amount in any one of the dialysate pressure, the venous pressure, and the arterial pressure during the above-mentioned operation is measured and compared with the reference value so as to judge the normality of the circuit connection.

The period of time until the clamp CL_{L4} is closed from the blood removal start operation, and a period of time during which the clamp CL_{L4} is closed are identical with those in the third embodiment.

A case where the circuit connection is normal in the fourth embodiment means a case where all portions indicated below are normal, and those portions are illustrated in FIG. 14. In other words, according to the fourth embodiment, in the hemodialysis apparatus whose normality is to be verified, the pressure variation amount in any one of the dialysate pressure, the venous pressure, and the arterial pressure during the above-mentioned operation is measured and compared with the reference value so as to surely judge the normality of the following portions.
a: The hemodialyzer D and the artery side blood line L3 are normally connected to each other.
b: The hemodialyzer D and the vein side blood line L4 are normally connected to each other.
c: The venous pressure measuring means M_{V} is normally connected.
d: The junction joint 2 is normally connected.
e: The clamp CL_{L5} is normally loaded.
f: The clamp CL_{L4} is normally loaded.
h: The arterial pressure measuring means M_{A} is normally connected.

It is desirable that the hemodialyzer D that brings blood into contact with the dialysate through the semipermeable membrane, and purifies the blood be of a hollow fiber type.

It is desirable that the dialysate supply line L1 that supplies the dialysate to the hemodialyzer D and the dialysate discharge line L2 that discharges the dialysate from the hemodialyzer D each be formed of a silicon tube.
Further, it is desirable that the artery side blood line L3 that allows the blood drawn from the patient to flow into the hemodialyzer D, and the vein side blood line L4 that returns the blood drained from the hemodialyzer D to the patient each be made of a flexible chemosynthesis material.

It is desirable that the first fluid feeding means P1 that feeds the dialysate to the hemodialyzer D and the second fluid feeding means P2 that absorbs the dialysate from the hemodialyzer D each be formed of a diaphragm pump or a duplex pump.
Further, it is desirable that the third fluid feeding means P3 that moves the dialysate into the blood circuit by reverse filtration through the hemodialyzer D, and removes the blood within the hemodialyzer D be formed of a reversible metering pump.
Further, it is desirable that the blood pump P4 that circulates the blood be formed of a roller tubing pump.

It is desirable that the vein side chamber C_{V} disposed on the vein side blood line L4, and the artery side chamber C_{A} disposed on the artery side blood line L3 each be made of a flexible chemosynthesis material.

Further, it is desirable that the overflow line L5 connected to the vein side chamber C_{V}, the venous pressure monitor line L6 also connected to the vein side chamber C_{V}, and the arterial pressure monitor line L7 connected to the artery side chamber C_{A} each be made of a flexible chemosynthesis material.

It is desirable that the dialysate pressure measuring means M_{T} that measures the dialysate pressure which is a fluid pressure of the dialysate flowing in the dialysate discharge line L2, the venous pressure measuring means M_{V} that measures the venous pressure which is a pressure within the vein side blood line L4, and the arterial pressure measuring means M_{A} that measures the arterial pressure which is a pressure within the artery side blood line L3 each be formed of a pressure transducer.

It is desirable that the clamp CL_{L4} disposed on the downstream side of the vein side chamber C_{V} and the clamp CL_{L5} disposed in the overflow line L5 each be formed of a solenoid type.

It is desirable that the control means G1 and the control means G2 each be formed of a computer (electronic computer).
Further, it is desirable that the judging means J1, the judging means J2, the judging means J3, and the judging means J4 each be formed of a computer (electronic computer).

## Claims

1. A hemodialysis apparatus, comprising:
a hemodialyzer (D) that brings blood into contact with a dialysate through a semipermeable membrane to purify the blood;
a dialysate supply line (L1) that supplies the dialysate to the hemodialyzer (D);
a dialysate discharge line (L2) that discharges the dialysate from the hemodialyzer (D);
an artery side blood line (L3) that allows the blood drawn from the patient to flow into the hemodialyzer (D);
a vein side blood line (L4) that returns the blood drained from the hemodialyzer (D) to the patient;
first fluid feeding means (P1) disposed in the dialysate supply line (L1);
second fluid feeding means (P2) disposed in the dialysate discharge line (L2);
reversible third fluid feeding means (P3) disposed in a bypass line that communicates an upstream side and a downstream side of any one or both of the first fluid feeding means (P1) and the second fluid feeding means (P2) with each other;
a blood pump (P4) disposed in the artery side blood line (L3);
a vein side chamber (C_{V}) disposed on the vein side blood line (L4);
an overflow line (L5) connected to the vein side chamber (C_{V});
a venous pressure monitor line (L6) connected to the vein side chamber (C_{V});
dialysate pressure measuring means (M_{T}) that is disposed in the dialysate discharge line (L2) and measures a dialysate pressure which is a fluid pressure of the dialysate flowing in the dialysate discharge line (L2);
venous pressure measuring means (M_{V}) that is connected to the venous pressure monitor line (L6), and measures a venous pressure which is a pressure within the vein side blood line (L4);
a clamp (CL_{L4}) disposed on a downstream side of the vein side chamber (C_{V});
a clamp (CL_{L5}) disposed in the overflow line (L5);
control means (G1) that closes the clamp (CL_{L5}) when a predetermined given period of time has elapsed since the start of priming, and operates the third fluid feeding means (P3) in a reverse filtration direction or a fluid removal direction, and then opens the clamp (CL_{L5}) when a predetermined given period of time has elapsed; and
judging means (J1) that compares a pressure variation amount in the dialysate pressure or the venous pressure for a period of time when the clamp (CL_{L5}) is closed with a predetermined reference value to judge normality of a circuit connection.

2. A hemodialysis apparatus, comprising:
a hemodialyzer (D) that brings blood into contact with a dialysate through a semipermeable membrane to purify the blood;
a dialysate supply line (L1) that supplies the dialysate to the hemodialyzer (D);
a dialysate discharge line (L2) that discharges the dialysate from the hemodialyzer (D);
an artery side blood line (L3) that allows the blood drawn from the patient to flow into the hemodialyzer (D);
a vein side blood line (L4) that returns the blood drained from the hemodialyzer (D) to the patient;
first fluid feeding means (P1) disposed in the dialysate supply line (L1);
second fluid feeding means (P2) disposed in the dialysate discharge line (L2);
reversible third fluid feeding means (P3) disposed in a bypass line that communicates an upstream side and a downstream side of any one or both of the first fluid feeding means (P1) and the second fluid feeding means (P2) with each other;
a blood pump (P4) disposed in the artery side blood line (L3);
a vein side chamber (C_{V}) disposed on the vein side blood line (L4);
an overflow line (L5) connected to the vein side chamber (C_{V});
a venous pressure monitor line (L6) connected to the vein side chamber (C_{V});
dialysate pressure measuring means (M_{T}) that is disposed in the dialysate discharge line (L2) and measures a dialysate pressure which is a fluid pressure of the dialysate flowing in the dialysate discharge line (L2);
venous pressure measuring means (M_{V}) that is connected to the venous pressure monitor line (L6), and measures a venous pressure which is a pressure within the vein side blood line (L4);
an artery side chamber (C_{A}) disposed on the artery side blood line (L3);
an arterial pressure monitor line (L7) connected to the artery side chamber (C_{A});
arterial pressure measuring means (M_{A}) that is connected to the arterial pressure monitor line (L7), and measures an arterial pressure which is a pressure within the artery side blood line (L3);
a clamp (CL_{L4}) disposed on a downstream side of the vein side chamber (C_{V});
a clamp (CL_{L5}) disposed in the overflow line (L5);
control means (G1) that closes the clamp (CL_{L5}) when a predetermined given period of time has elapsed since the start of priming, and operates the third fluid feeding means (P3) in a reverse filtration direction or a fluid removal direction, and then opens the clamp (CL_{L5}) when a predetermined given period of time has elapsed; and
judging means J2 that compares a pressure variation amount in any one of the dialysate pressure, the venous pressure, and the arterial pressure for a period of time when the clamp (CL_{L5}) is closed with a predetermined reference value to judge normality of a circuit connection.

3. The hemodialysis apparatus according to claim 1, wherein the control means (G1) as claimed in claim 1 is replaced with control means (G2) that closes the clamp (CL_{L4}) when a predetermined given period of time has elapsed from a blood removal start operation, and operates the third fluid feeding means (P3) in the fluid removal direction or the reverse filtration direction, and then opens the clamp (CL_{L4}) when a predetermined given period of time has elapsed, and
wherein the judging means (J1) as claimed in claim 1 is replaced with judging means (J3) that compares a pressure variation amount in the dialysate pressure or the venous pressure for a period of time when the clamp (CL_{L4}) is closed with a predetermined reference value to judge normality of a circuit connection.

4. The hemodialysis apparatus according to claim 2, wherein the control means (G1) as claimed in claim 2 is replaced with control means G2 that closes the clamp (CL_{L4}) when a predetermined given period of time has elapsed from a blood removal start operation, and operates the third fluid feeding means (P3) in the fluid removal direction or the reverse filtration direction, and then opens the clamp (CL_{L4}) when a predetermined given period of time has elapsed, and
wherein the judging means (J2) as claimed in claim 2 is replaced with judging means (J4) that compares a pressure variation amount in any one of the dialysate pressure, the venous pressure, and the arterial pressure for a period of time when the clamp (CL_{L4}) is closed with a predetermined reference value to judge normality of a circuit connection.
